Europäisches Patentamt

European Patent Office

Office européen des brevets

(19)

(11) Publication number: **0 218 352 B1**

# EUROPEAN PATENT SPECIFICATION

(45) Date of publication of patent specification: **10.07.91**

(51) Int. Cl.5: **C12N 13/00**, C12N 1/06,
//(C12P21/00,19:40)

(21) Application number: **86306599.1**

(22) Date of filing: **27.08.86**

(54) **A process for treating protista cells.**

(30) Priority: **29.08.85 JP 190612/85**

(43) Date of publication of application:
**15.04.87 Bulletin 87/16**

(45) Publication of the grant of the patent:
**10.07.91 Bulletin 91/28**

(84) Designated Contracting States:
**DE FR GB SE**

(56) References cited:
**EP-A- 0 137 504**
**US-A- 4 292 408**

**SCIENCE, vol. 213, 31st July 1981, pages 505-513, AAAS; M.W. BERNS et al.: "Laser microsurgery in cell and developmental biology"**

**CHEMICAL ABSTRACTS, vol. 104, no. 5, February 1986, page 298, abstract no. 31399g, Columbus, Ohio, US; & JP-A-60 164 487 (HITACHI LTD.) 27-08-1985**

(73) Proprietor: **NIPPON ZEON CO., LTD.**
**6-1, 2-chome, Marunouchi, Chiyoda-ku Tokyo(JP)**

Proprietor: **ISHIKAWAJIMA-HARIMA JUKOGYO KABUSHIKI KAISHA**
**2-1, Ote-machi 2-chome Chiyoda-ku Tokyo 100(JP)**

(72) Inventor: **Ito, Akitoshi**
**26-1-405 Kajiwara-2-chome Kamakura-shi(JP)**
Inventor: **Takahashi, Kazumasa**
**7-14 Izumicho-1-chome Hoya-shi(JP)**
Inventor: **Shiozaki, Shozo**
**26-1-202 Kajiwara-2-chome Kamakura-shi(JP)**
Inventor: **Morikawa, Shigeru**
**1059 Totsukacho Totsuka-ku Yokohama(JP)**
Inventor: **Katayama, Akira**
**26-22 Morigaoka-2-chome Isogo-ku Yokohama(JP)**

Inventor: **Udagawa, Takeshi**
**13-1-409 Kajigaya-2-chome**
**Takatsu-ku Kawasaki-shi(JP)**
Inventor: **Kobayashi, Takeshi**
**Shintoyosuryo 3-23 Toyosu-3-chome**
**Koto-ku Tokyo(JP)**

(74) Representative: **Ellis, John Clifford Holgate et**
**al**
**MEWBURN ELLIS & CO. 2/3 Cursitor Street**
**London EC4A 1BQ(GB)**

## Description

This invention relates to a process for treating protista cells having a useful substance accumulated therein, more particularly a process for recovering the useful substance in the cells by irradiating the cells with laser beam.

In the past, investigations relating to the production of various useful substances by fermentation methods using protista cells such as bacteria and yeasts have been carried out. Specific examples of such useful substances include, for example, enzymes and proteins such as trypsin, amylase, cellulase, lysozyme, ribonuclease, cytochrome, insulin, pepsin and single cell proteins (SCP); amino acids such as lysine, glutamic acid, leucine, tryptophan, arginine, homoserine and methionine; nucleic acids such as nucleosides, nucleotides, oligonucleotides, DNA and RNA; vitamins such as vitamin $B_1$, vitamin $B_2$, vitamin $B_6$ and vitamin H; and biologically active substances such as S-adenosyl-L-methionine (hereinafter referred to as SAM in some cases), S-adenosylhomocysteine, glutathione, interferon and interleukin.

Previously known treatment processes for extracting these useful substances from cells are, for example, a mechanical grinding process using, for example, a mortar ball mill or homogenizer; an ultrasonication process comprising bacteriolysis by autolysis; a process comprising treatment with an agent such as perchloric acid, sulfuric acid, formic acid, acetic acid, ethyl acetate, acetone or toluene; and a process comprising treatment with a cell wall-degrading enzyme.

However, the mechanical process and the ultrasonic process are disadvantageous in that since destruction of cell walls results in discharge of all substances in cells, a very troublesome procedure is required for thereafter selectively separating and purifying a useful substance, for example, only a protein having a molecular weight in a specific range. The process comprising bacteriolysis by autolysis and the process comprising treatment with a cell wall-degrading enzyme are applicable only to limited kinds of protista. Furthermore, the treatment with an agent such as perchloric acid permits substantially complete extraction of a useful substance in cells but has been disadvantageous in that it requires a subsequent neutralization step or results in decomposition of the useful substance or increases its toxicity.

Berns et al, Science, (1981), 213,505, disclose various uses of laser microbeams for laser microsurgery in cell and development biology. The principles of selective damage and their application to detailed studies of cell behaviour are discussed.

Recently, a process has been developed for incorporating a biopolymer such as gene or protein into viable cells of microorganisms by irradiating the viable cells with a laser beam (for example, EP-A-0137504). However, this process aims merely at incorporating an extracellular substance into viable cells and does not address the problem of selectively extracting from cells a useful substance present in such cells.

Accordingly, we conducted research directed to finding a process for treating cells which can solve the above-mentioned problems encountered in the conventional treatments of cells, which comprises simpler steps, and which is applicable to various kinds of protista. As a consequence, we found that a useful substance can be discharged easily from protista cells by irradiation of the cells with a laser beam.

The invention provides a process for treating protista cells which makes it possible to extract a useful substance from a variety of protista cells by a simple procedure. Such a process of this invention can be achieved by irradiating protista cells having a useful substance therein with a laser beam to discharge the useful substance from the cells.

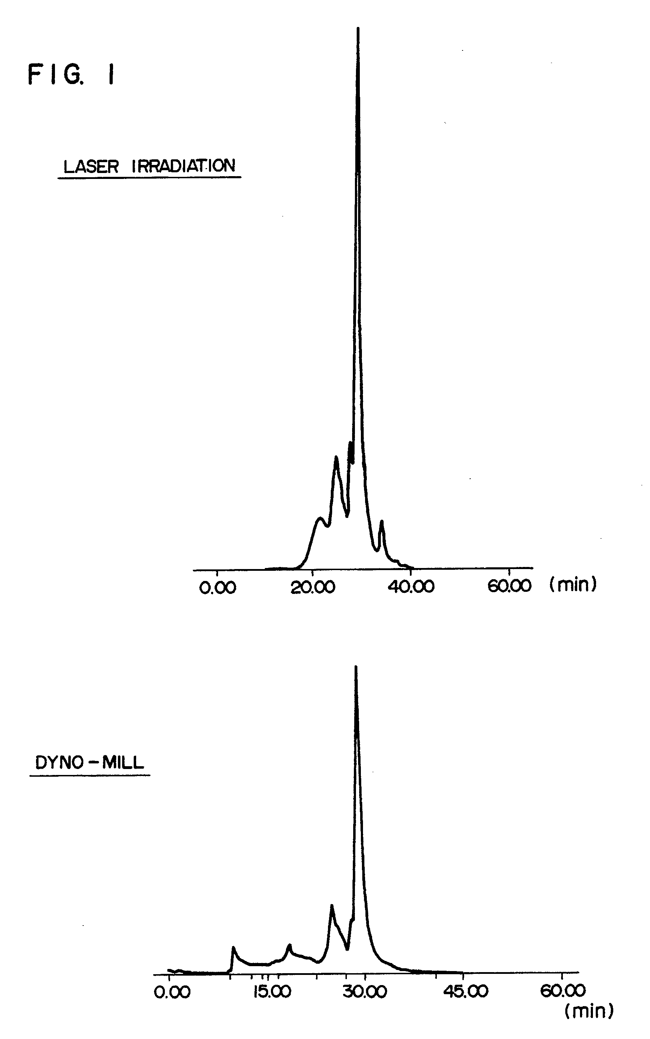

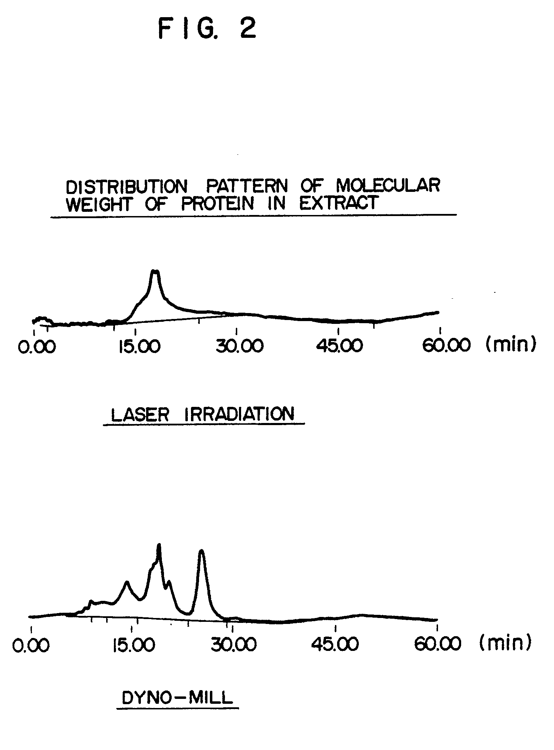

The results achievable by performance of a process embodying the invention are illustrated in Figs. 1 and 2, which show analysis charts of HPLC of the proteins obtained in Examples 1 and 6 (see later), respectively.

The invention is applicable to a wide spectrum of protista, ranging from higher protista to lower protista. Specific examples of the protista include bacteria such as Escherichia, Pseudomonas, Bacillus, Brevibacterium, Corynebacterium, Serratia, Citrobacter, Streptococcus, Lactobacillus and Staphylococcus; fungi such as Aspergillus, Penicillium, Mucor, Rhizopus, Gibberella, Monascus, Neurospora, Botrytis, Cladospolium and Fusarium; yeasts such as Saccharomyces, Candida, Pichia, Hansenula, Schizosaccharomyces, Lipomyces and Rhodotorula; Actinomycetes such as Streptomyces, Nocardia and Actinomyces; and Bacidiomycetes such as Lentinus, Trametes, Coprinus and Pycnoporus.

Although the structures and strengths of cell walls of these protista are different, useful substances in the cells can be discharged from the cells by inflicting an adequate injury on the cell walls by properly varying the wavelength and intensity of a laser beam directed at the cells. The process is applicable to a higher or a lower protista.

The laser beam used in a process of this invention usually has a specific wavelength in the range of from the ultraviolet region to the infrared region, and its output mode may be either CW (continuous wave) or pulse. When the useful substance is unstable to heat, the laser beam is preferably pulsed. In this case, a rise of temperature due to light absorption of the substance can be prevented by a non-irradiation time between pulses, so that even a useful substance unstable to heat becomes extractable in a stable state.

The laser beam used includes solid-state lasers such as a YAG laser, glass laser or ruby laser, a dye laser, and gas lasers such as an excima laser or argon ion laser. Furthermore, lasers pumped by other lasers such as a YAG laser pumped-dye laser, excima laser pumped-dye laser or argon ion laser pumped-dye laser may be used.

The treatment of cells with a laser beam is usually carried out by suspending the cells in a liquid medium for example, water, a buffer solution or an organic solvent such as an alcohol, ketone, ester, ether or hydrocarbon, and irradiating the resulting suspension with a laser beam.

The mode of the treatment with the laser beam may properly be selected depending on the purpose, and any of the batch mode, semicontinuous mode and continuous mode can be employed.

For example, in the case of the batch mode, it is sufficient that the suspension of protista cells are irradiated with the laser beam. In the case of the semi-continuous mode or the continuous mode, the treatment is carried out by feeding the suspension of protista cells to a laser beam irradiation zone intermittently or continuously. The method for feeding the cell suspension is not critical and includes, for instance, a method comprising feeding the suspension from an upper part of the laser beam irradiation zone and removing it from the lower part; a method comprising feeding the suspension from a lower part and removing it from the upper part; and a method comprising passing the suspension through the laser beam irradiation zone transversely.

As a process for treating a large amount of cells efficiently, the semicontinuous mode or the continuous mode is preferred among these treatment modes, and according to them, the efficiency of treatment can be raised by recycling the cell suspension after the treatment to the laser beam irradiation zone. In the recycling, it is also possible to separate cells by a conventional method such as centrifugation, then prepare a suspension thereof again and again feed it to the irradiation zone.

The content of cells in the cell suspension is usually 0.01 to 50% by weight, preferably 0.1 to 20% by weight in terms of the weight of the wet cells. Although the temperature of the cell suspension is not critical so long as it causes no denaturation of a desired useful substance, it is usually $0^\circ$ to $100^\circ$ C, preferably $0^\circ$ to $50^\circ$ C. The irradiation time of the laser beam is usually tens of seconds to about 10 hours, preferably 1 minute to 2 hours.

Although the irradiation dose of the laser beam is varied depending, for example, on the kind of cells and the kind of substance to be extracted, it can easily be determined by carrying out a simple preliminary experiment.

As a method for recovering a desired useful substance from the cell suspension after the treatment, a conventional method may be used. The recovery method includes, for example, a method comprising directly obtaining the useful substance by extraction with a solvent, and a method comprising removing cell debris by centrifugation or the like and recovering the useful substance from the thus obtained supernatant by a technique such as ion-exchange chromatography, gel filtration, electrophoresis, solvent precipitation or salting-out.

Thus, according to this invention, intracellular useful substances can be extracted from various kinds of protista cells very efficiently by a simple procedure.

EXAMPLES

This invention is now explained in more detail below with reference to Examples. In the following Examples, SAM was determined by using a high-performance liquid chromatography (hereinafter abbreviated as HPLC) (Model TRI-V mfd. by Japan Spectroscopic Co., Ltd., Column: TSK-SP-2SW, Detector: UV 258 nm). Protein was determined quantitively by the Lowry method [see J. Biol. Chem. 193, 265 - 275 (1951)]. In order to investigate the distribution pattern of the molecular weight of protein, measurement was carried out by using the above-mentioned HPLC (column: Asahipack GS-510, Detector: UV 280 nm).

Example 1

Saccharomyces cerevisiae IFO-2044 was cultured in the medium of Schlenk, F. et al. [see J. Biol. Chem. 229, 1037 (1957)] and 1 g (wet weight) of the S-adenosylmethionine (SAM)-containing cells thus obtained were suspended in 99 ml of distilled water. Then, 3ml of the resulting suspension was placed in a quartz cuvette and irradiated with a YAG laser pumped - dye laser system (wavelength 266 nm, 15mJ/pulse, 10Hz) for 10 minutes with stirring by means of a stirrer. The irradiated suspension was centrifuged to remove a cell debris, and the amount of SAM in the supernatant thus obtained was measured by HPLC to be 42.9 mg.

In 4 ml of 1.5N perchloric acid was suspended 1 g (wet weight) of the same cultured cells as described above, and subjected to shaking extraction at 37°C for 1 hour, after which a cell debris was removed from the mixture by centrifugation and the supernatant was adjusted to pH 5.0 by addition of potassium hydrogen-carbonate. After the resulting precipitate of potassium perchlorate was removed by centrifugation and filtration, the amount of SAM in the supernatant thus treated was measured and found to be 55.0 mg.

As a result, the percentage of extraction of SAM by laser irradiation was calculated to be 78.0% from the equation:

$$
\text{SAM extraction percentage (\%)} = \frac{\text{Amount (mg) of SAM extracted by laser beam irradiation}}{\text{Amount (mg) of SAM extracted with perchloric acid}} \times 100
$$

Further, the protein concentration in the supernatant obtained by the procedure described above was measure and found to be 0.46 mg. For comparison, a suspension of 1 g of wet cells in 99 ml of 0.2M patassium phosphate buffer (pH 7.5) containing 5mM dithiothreitol (hereinafter abbreviated as DTT) was ground and stirred by means of a DYNO-MILL (mfd. by Willy. A. Bachofen A.G.) at 4,500 r.p.m. for 11 minutes by using glass beads (0.25 to 0.5 mm), to disrupt the cells. The suspension was centrifuged to remove the cell debris and the glass beads, and the protein concentration in the cell-free extract thus obtained was measured to be 0.97 mg. That is to say, protein was extracted by the laser irradiation in an amount of 47% of the amount of protein extracted by means of the DYNO-MILL.

HPLC analysis was carried out in order to investigate the distribution pattern of the molecular weight of protein in each extract. An analytical chart is shown in Fig. 1. It can be seen that the molecular weight distribution in the case of the laser irradiation is kept to within a specific range, as compared with that observed in the case of the extraction by means of the DYNO-MILL.

Example 2

Escherichia coli ATCC-14948 was inoculated into a liquid medium consisting of 1g/dl of glucose, 1.5g/dl of peptone, 0.3g/dl of yeast extract, 0.3g/dl of $K_2HPO_4$, 0.2g/dl of NaCl and 0.02g/dl of $MgSO_4 \cdot 7H_2O$ which had been adjusted to pH 7.0 and sterilized by heating, and the cells were subjected to shaking culture at 28°C for 16 hours. The culture broth was centrifuged and 1 g (wet weight) of the cells thus obtained were suspended in 99 ml of 0.02M potassium phosphate buffer (pH 7.5) containing 5mM DTT. Then, 3 ml of the resulting suspension was placed in a quartz cuvette and irradiated with a YAG laser pumped - dye laser system (wavelength 280 nm, 1mJ/pulse, 10 Hz) for 10 minutes with stirring by means of a stirrer. Subsequently, the suspension thus treated was centrifuged to remove cell debris, and the protein concentration in the cell-free extract thus obtained was measured and found to be 77 mg/g wet cell.

On the other hand, 5 g (wet weight) of the same cells as described above were suspended in 10 ml of 0.02M potassium phosphate buffer (pH 7.5) containing 5mM DTT, and the resulting suspension was subjected to ultrasonic cell disintegration at 2° to 15°C for 10 minutes. Next, the suspension thus treated was centrifuged to remove cell debris, and the protein concentration in the cell-free extract thus obtained was measured and found to be 78 mg/g wet cell.

From this result, the percentage of extraction of protein by the laser irradiation was calculated and found to be 98.7% based on the protein extracted by the ultrasonic cell disintegration.

Example 3

Penicillium oxalicum IFO-5748 was inoculated into a liquid medium consisting of 5g/dl of glucose, 0.5g/dl of peptone, 0.1g/dl of yeast extract, 2.0g/dl of $KH_2PO_4$, 0.1g/dl of $K_2HPO_4$ and 0.02g/dl of $MgSO_4 \cdot 7H_2O$ which had been adjusted to pH 6.5 and sterilized by heating, and the cells were subjected to shaking culture at 28°C for 48 hours. The culture broth was centrifuged and 1 g (wet weight) of the cells thus obtained were suspended in 99 ml of 0.02M potassium phasphate buffer (pH 7.5) containing 5mM

EP 0 218 352 B1

DTT. Then, 3 ml of the resulting suspension was placed in a quartz cuvette and irradiated with a YAG laser pumped - dye laser system (wavelength 266 nm, 15mJ/pulse, 10Hz) for 15 minutes with stirring by means of a stirrer. Subsequently, the suspension thus treated was centrifuged to remove cell debris, and the protein concentration in the cell-free extract thus obtained was measured and found to be 34 mg/g wet cell.

On the other hand, 1g (wet weight) of the same cells as described above were suspended in 9ml of 0.02 M potassium phosphate buffer containing 5mM DTT, after which 1 g of quartz sand was added thereto and the resulting mixture was sufficiently ground in a mortar. Next, the mixture was centrifuged to remove cell debris and the quartz sand, and the protein concentration in the cell-free extract thus obtained was measured and found to be 36 mg/g wet cell.

From this result, the percentage of extraction of protein by laser irradiation was calculated and found to be 94.4% based on the protein extracted by the grinding.

Example 4

Streptomyces hygroscopicus IFO-3192 was inoculated into a liquid medium consisting of 1g/dl of peptone, 0.5g/dl of meat extract, 0.1g/dl of yeast extract and 0.5g/dl of NaCl which had been adjusted to pH 7.0 and sterilized by heating, and the cells were subjected to shaking culture at 34°C for 72 hours. The culture broth was centrifuged and 1 g (wet weight) of the cells thus obtained were suspended in 99 ml of 0.02M potassium phosphate buffer (pH 7.5) containing 5mM DTT. Then, 3ml of the resulting suspension was placed in a quartz cuvette and irradiated with a YAG laser pumped - dye laser system (wavelength 266 nm, 15mJ/pulse, 10Hz) for 10 minutes with stirring by means of a stirrer. Subsequently, the suspension thus treated was centrifuged to remove cell debris, and the protein concentration in the cell-free extract thus obtained was measured and found to be 29 mg/g wet cell.

On the other hand, 5 g (wet weight) of the same cells as described above were suspended in 10 ml of 0.02M potassium phosphate buffer containing 5mM DTT and the resulting suspension was subjected to ultrasonic cell disintegration at 2° to 15°C for 10 minutes. Next, the suspension thus treated was centrifuged to remove cell debris, and the protein concentration in the cell-free extract thus obtained was measured and found to be 32.1 mg/g wet cell.

From this result, the percentage of extraction of protein by laser irradiation was calculated and found to be 90.3% based on the protein extracted by the ultrasonic cell disintegration.

Example 5

In 99 ml of distilled water was suspended 1 g (wet weight) of Saccharomyces cerevisiae cells obtained by culture in the same manner as in Example 1, and the resulting suspension was passed through an upright quartz flow cuvette (2 x 10 x 100 mm) under irradiation with a YAG laser pumped - dye laser system (wavelength 266 nm, 15mJ/pulse, 10Hz), from the lower part at a flow rate of 0.4 ml/min by using a metering pump. The treated suspension which flowed from the upper part of the flow cuvette was recovered and then centrifuged to remove cell debris, and the amount of SAM in the supernatant thus obtained was measured by HPLC and found to be 38.9 mg.

The extraction percentage in the case of this process was calculated and found to be 70.7% from the fact that in Example 1, the SAM content of the cells was 55.0 mg.

Example 6

Pycnoporus coccineus IFO 6489 was inoculated into a liquid medium consisting of 5g/dl of glucose, 0.5g/dl of peptone, 0.1g/dl of yeast extract, 0.2g/dl of $KH_2PO_4$, 0.1g/dl of $K_2HPO_4$ and 0.02g/dl of $MgSO_4 \cdot 7H_2O$ which had been adjusted to pH 6.5 and sterilized by heating, and the cells were subjected to shaking culture at 28°C for 72 hours. The culture broth was filtered and 1 g (wet weight) of the cell's thus obtained were suspended in 99 ml of 0.02M potassium phosphate buffer (pH 7.5) containing 5mM DTT. Then, 3ml of the resulting suspension was placed in a quartz cuvette and irradiated with a YAG laser pumped - dye laser system (wavelength 280nm, 12mJ/pulse, 10Hz) for 15 minutes with stirring by means of a stirrer. Subsequently, cell debris was removed from the thus treated suspension by filtration, and the protein concentration in the cell-free extract thus obtained was measured and found to be 8.6 mg/g wet cell.

On the other hand, 1g (wet weight) of the same cells as described above were suspended in 99 ml of 0.02M potassium phosphate buffer containing 5mM DTT, and the resulting suspension was treated by means of a DYNO-MILL (mfd. by Willy. A. Bachofen A.G.) at 4°C at 4,500 r.p.m. for 11 minutes by using glass beads for DYNO-MILL (0.25 to 0.5 mm), to grind the cells. Next, the suspension treated was

6

centrifuged to remove cell debris and the glass beads, and the protein concentration in the cell-free extract thus obtained was measured and found to be 9.8 mg/g wet cell. From this result, the percentage of extraction of protein by laser irradiation was calculated and found to be 87.7% based on the protein extracted by the grinding by means of the DYNO-MILL.

Further, in order to investigate the distribution pattern of the molecular weight of protein in each extract, HPLC analysis was carried out by use of column of the gel filtration type in the same manner as in Example 1. An analysis chart obtained is shown in Fig. 2. Clearly, the molecular weight distribution in the case of the laser irradiation is observed in a specific range, as compared with that observed in the case of the extraction by means of DYNO-MILL.

## Claims

1. A process for treating protista cells containing a useful substance which comprises irradiating the protista cells with a laser beam so as to selectively release from the protista cells material of a molecular size not significantly larger than that of the useful substance while unwanted material of a significantly large size is retained within the cells.

2. A process according to claim 1, wherein the laser beam is continuous-wave or pulse.

3. A process according to claim 1 or 2, wherein said protista cells are used in the form of a suspension in a vehicle.

4. A process according to claim 3, wherein the content of said protista cells in the suspension is 0.01 to 50 wt%.

5. A process according to claim 3 or 4, wherein said vehicle is water, a buffer solution or an organic solvent.

6. A process according to claim 3, 4 or 5, wherein the temperature of said suspension is 0° to 100°C.

7. A process according to any preceding claim, wherein said protista is a bacterium, fungus, yeast, actinomycete or basidiomycete.

8. A process according to any preceding claim, wherein said irradiation with laser beam is carried out for a period in a range of from tens of seconds to 10 hours.

9. A process according to any preceding claim, wherein said useful substance is an enzyme, protein, amino acid, nucleic acid, vitamin or biologically active substance.

## Revendications

1. Procédé de traitement de cellules de protiste contenant une substance utile, qui comporte l'irradiation des cellules de protiste avec un faisceau laser, de façon à libérer sélectivement des cellules de protiste une matière présentant une taille moléculaire non significativement supérieure à celle de la substance utile, pendant que la matière non recherchée, de taille significativement grande, est retenue à l'intérieur des cellules.

2. Procédé conforme à la revendication 1, dans lequel le faisceau laser fonctionne en mode continu ou par impulsions.

3. Procédé conforme à la revendication 1 ou 2, dans lequel lesdites cellules de protiste dont utilisées sous la forme d'une suspension dans un véhicule.

4. Procédé conforme à la revendication 3, dans lequel la teneur de la suspension en lesdites cellules de protiste vaut de 0,01 à 50 % en poids.

5. Procédé conforme à la revendication 3 ou 4, dans lequel ledit véhicule est de l'eau, une solution tampon ou un solvant organique.

6. Procédé conforme à la revendication 3, 4 ou 5, dans lequel la température de ladite suspension vaut de 0 à 100° C.

7. Procédé conforme à l'une quelconque des revendications précédentes, dans lequel ledit protiste est une bactérie, un champignon inférieur, une levure, un actinomycète ou un basidiomycète.

8. Procédé conforme à l'une quelconque des revendications précédentes, dans lequel ladite irradiation avec le faisceau laser est effectuée pendant une durée comprise entre une dizaine de secondes et dix heures.

9. Procédé conforme à l'une quelconque des revendications précédentes, dans lequel ladite substance utile est une enzyme, une protéine, un acide aminé, un acide nucléique, une vitamine ou une substance biologiquement active.

**Ansprüche**

1. Verfahren zur Behandlung von Protisten-Zellen, die einen nutzbaren Stoff enthalten, umfassend die Bestrahlung der Protisten-Zellen mit einem Laserstrahl, so daß aus den Protisten-Zellen selektiv Material einer Molekülgröße, die nicht signifikant größer ist als die des nutzbaren Stoffes freigesetzt wird, während unerwünschtes signifikant größeres Material in den Zellen verbleibt.

2. Verfahren nach Anspruch 1, wobei der Laserstrahl kontinuierlich oder gepulst ist.

3. Verfahren nach Anspruch 1 oder 2, wobei die Protisten-Zellen in Form einer Suspension in einem Bindemittel verwendet werden.

4. Verfahren nach Anspruch 3, wobei der Gehalt an Protisten-Zellen in der Suspension 0,01 bis 50 Gew.-% beträgt.

5. Verfahren nach Anspruch 3 oder 4, wobei das Bindemittel Wasser, eine Pufferlösung oder ein organisches Lösungsmittel ist.

6. Verfahren nach Anspruch 3, 4 oder 5, wobei die Temperatur der Suspension 0 bis 100° C ist.

7. Verfahren nach einem der vorangehenden Ansprüche, wobei der Protist ein Bakterium, ein Pilz, eine Hefe, ein Actinomycet oder ein Basidiomycet ist.

8. Verfahren nach einem der vorangehenden Ansprüche, wobei die Bestrahlung mit einem Laserstrahl für einen Zeitraum im Bereich von Zehntelsekunden bis zu 10 Stunden durchgeführt wird.

9. Verfahren nach einem der vorangehenden Ansprüche, wobei der nutzbare Stoff ein Enzym, Protein, eine Aminosäure, Nucleinsäure, ein Vitamin oder eine biologisch wirksame Substanz ist.

# F I G. I

LASER  IRRADIATION

DYNO - MILL

# F I G. 2

DISTRIBUTION PATTERN OF MOLECULAR WEIGHT OF PROTEIN IN EXTRACT

LASER IRRADIATION

DYNO-MILL